# EUROPEAN PATENT SPECIFICATION

(11) **EP 1 420 857 B1**
(45) Date of publication and mention of the grant of the patent: **06.05.2009**
(21) Application number: 01982627.0
(22) Date of filing: 24.10.2001
(51) Int. Cl.: A61N 5/06

(54) **PHOTOTHERAPEUTICAL APPARATUS**
PHOTOTHERAPEUTISCHER APPARAT
APPAREIL PHOTOTHERAPEUTIQUE

(30) Priority: 10.08.2001 HU 0103279
(43) Date of publication of application: 26.05.2004
(73) Proprietor: Rhinolight Ltd., 6726 Szeged (HU)
(72) Inventor: Kemény, Lajos, 6721 Szeged (HU); Dobozy, Attila, 6721 Szeged (HU); Bor, Zsolt, 6726 Szeged (HU); Rácz, Béla, 6723 Szeged (HU); Szabó, Gábor, 6722 Szeged (HU); Ignácz, Ferenc, 6449 Mélykút (HU)
(74) Representative: Varnai, Aniko
(86) International application number: PCT/HU2001/000102
(87) International publication number: WO 2003/013653

(56) References cited:
- EP-A- 0 277 505
- EP-A- 0 435 506
- WO-A-02/13905
- WO-A-96/36396
- WO-A-98/22184
- DE-C- 19 954 710
- DE-U- 9 104 584
- GB-A- 2 340 926
- US-A- 1 616 722
- US-A- 1 677 016
- US-A- 1 782 906
- US-A- 1 800 277
- US-A- 2 227 422
- US-A- 5 292 346
- US-A- 5 344 433
- US-A- 5 855 595
- US-A- 5 925 034

## Description

The subject matter of this invention is a phototherapeutical apparatus.

This invention relates to an apparatus, which is applicable for the treatment and prevention of common inflammatory diseases of the nasal mucous membrane and paranasal sinuses such as allergic rhinitis (hay fever), vasomotor rhinitis, non-allergic eosinophilic rhinitis, chronic sinusitis (inflammation in the paranasal sinuses), or nasal polyps with ultraviolet light.

Rhinitis is an inflammatory disorder of the nasal mucous membrane, which is characterised by nasal itch, sneeze, nose running, nasal blockage, and rarely by loss of smelling. The inflammation of the nasal mucous membrane is frequently associated with the inflammation of the paranasal sinuses (rhinosinusitis, chronic sinusitis). As a consequence of the frequent and persistent inflammation of the mucous membrane hyperproliferative lesions, the so-called polyps develop on the mucous membrane.

One characteristic disease is the allergic rhinitis (hay fever).

The allergic rhinitis or hay fever is the most frequent allergic disease affecting 10-20% of the population. The number of patients with allergic rhinitis especially in the well developed industrial countries increase very rapidly in the last few years. Because of the high number of patients the direct and indirect costs of the treatment are huge.

Although the hay fever is not a very severe disease, its unpleasant symptoms largely worsen the quality of life. Hay fever is frequently associated with allergic conjunctivitis, and sometimes by general symptoms. The symptoms last only for a few months in some patients (seasonal rhinitis), while in others it last in the whole year (perennial rhinitis).

For the development of the symptoms the allergen-induced specific IgE binding to the surface of mast cells, then after repeated exposure with the antigen the activation of cells possessing IgE receptors are responsible.

As a result of this activation histamin and other preformed mediators will be released from the mast cells, and in these cells new inflammatory mediators will be produced attracting further inflammatory cells into the mucous membrane (Howarth PH, Salagean M, Dokic D: Allergic rhinitis: not purely a histamine-related disease. Allergy 55: 7-16, 2000).

The treatment of the disease is not solved. To block the released mediators from the increased number of inflammatory cells in the nasal mucous membrane, which are responsible for the clinical symptoms, antihistamines are used locally or systemically; to inhibit the mediator release sodium cromoglycate is available and to block new mediator synthesis corticosteroids are used locally or systemically. In special circumstances desensitizing therapy might be introduced. Although the pathogenesis of the development of the clinical symptoms is already well known in general, with the available drugs for the treatment of this disease complete symptom-free state might not be reached frequently, therefore, every new method for the treatment of this disease has a great medical significance.

Another characteristic disease is vasomotor rhinitis.

Vasomotor rhinitis is an inflammatory disorder of the nasal mucous membrane with unknown origin, which might be induced by different unspecific factors. The clinical symptoms are largely similar to that of allergic rhintitis; permanent nasal blockage, nasal itch, sneeze, nose running, and rarely loss of smelling can be found. For the symptoms mastocyte-activating mediators are responsible, which are released from the nerve endings of the nasal mucous membrane upon irritative effects.

Another characteristic disease is the nonallergic eosinophilic rhinitis.

This disease is characterised by the high number of eosinophils in the nasal secretions and by the lack of allergic origin. The disease is frequently associated with the development of nasal polyps, the hyperproliferative condition of the nasal mucous membrane. The clinical symptoms are the same as in allergic rhinitis.

Another diseases are rhinosinusitis and sinusitis.

The inflammation of the paranasal sinuses is frequently associated with the inflammatory condition of the nasal mucous membrane (nasosinusitis), but isolated inflammatory condition of the paranasal sinuses is also a frequent disease (sinusitis). This disease has oft an allergic origin, although its exact cause remains frequently unknown. The treatment is not solved, but usually the same therapy is used as for rhintitis.

Ultraviolet light is used for more than 20 years for the treatment of allergic and auto-immune skin diseases. Ultraviolet light inhibits the antigen-induced cellular immune response, and is able to induce tolerance (Streilein JW, Bergstresser PR: Genetic basis of ultraviolet-B on contact hypersensitivity. Immunogenetics 27: 252-258, 1988).

The immune reaction suppressive effect of ultraviolet light is based on its inhibitory effect on the antigen presentation, and on the induction of T-cell apoptosis. Irradiation of the skin with ultraviolet-B light (280 nm - 320 nm), or with ultraviolet-A light (320 nm - 400 nm) on a previously psoralen-photosensitized skin, inhibits the immunological processes in the skin. For the treatment of skin diseases there are a number of phototherapeutical devices available.

At present there are many different ultraviolet light sources available.

These light sources might be grouped based on different aspects. (operational principle, output energy or power, operating in impulse mode or in continuous mode, emitting monochromatic or polychromatic light, etc).

Broadband ultraviolet light sources were used earlier for the treatments with ultraviolet light (broadband UVB, BB-UVB), in the last years more efficacious narrow-band ultraviolet light sources (narrow-band, NB-UVB) came into general use (Degitz K, Messer G, Plewig G, Röcken M: Schmalspektrum-WB 311 nm versus Breitspektrum-UVB. Neue Entwicklungen in der Phototherapie. Hautarzt 49: 795-806, 1998).

Based on our previous investigations on psoriatic patients, the 308 nm xenon chloride excimer laser is more effective in the treatment than the NB-UVB one (Bónis B, Kemény L, Dobozy A, Bor Zs, Szabó G, Ignácz F: 308 nm UVB excimer laser for psoriasis. Lancet 35: 1522, 1997; Kemény L, Bónis B, Dobozy A, Bor Z, Szabo G, Ignacz F: 308-nm excimer laser therapy for psoriasis. Arch Dermatol. 137: 95-96, 2001).

In the phototherapy a significant progress was achieved by the appearance of the ultraviolet light delivering optical systems. Such an ultraviolet light delivering phototherapeutical system with fibre optic is used in the Saalmann Cup instrument, in which the concentrated ultraviolet light is conducted into a fibre optic cable. Therefore, it is suitable for the treatment of smaller lesions of the skin or mucous membrane (Taube KM, Fiedler H: Hochkonzentrierte UV Bestrahlung kleiner Hautbezirke mit einem neuen Punktstrahler. Grundlagen und klinische Ergebnisse. Deutsche Dermatologe, 10: 1453, 1992).

This system is, however, unsuitable to be introduced into smaller cavernous organs because of the large diameter of the area, which is in contact with the skin or mucous membrane. It cannot be introduced into smaller cavernous organs, such as the nasal cavity, because of the thickness of the fibre optic cable. Only the cavernous organs might be treated with it, in which the distal end of the fibre optic cable and the area to be treated can be controlled by eye (i.g. oral cavity). It is also unsuitable for the treatment of body areas, which cannot be controlled by eye (nasal and paranasal mucous membrane, gastrointestinal or urogenital mucous membrane).

There are a number of light delivery systems to conduct and to emit ultraviolet light of ultraviolet light emitting laser light sources. The 308 nm xenon chloride excimer laser light delivered by fibre optic cable is suitable for the treatment of smaller cavities, like for the cleaning of root canal by ablation (Folwaczny M, Mehl A, Haffner C, Hickel R: Substance removal on teeth with and without calculus using 308 nm XeCl excimer laser radiation. An in vitro investigation. J Clin Periodontol 26: 306-12, 1999). It is suitable to treat atherosclerosis by treating the blood vessel walls (U.S. Patent 4,686,979), or to enhance the cardiac oxygenisation with transmyocardial laser revascularisation (U.S. Patent 5,976,124), or inhibiting neovascularisation during angioplasty by destroying myocardial cells (U.S. Patent 5,053,033).

It is an important common feature of these systems, that the high-energy ultraviolet light at the end of the light delivering system reaches small areas of only a few hundred microns, where the mechanism of action is to induce breakage in the chemical bondage, thereby a tissue damaging ablative effect. Using a multitude of small fibre optic cables the treatment of larger skin lesion has become possible (U.S. 6,071,302; WO9607451, Asawanonda P, Anderson RR, Chang Y, Taylor CR: 308-nm excimer laser for the treatment of psoriasis: a dose-response study. Arch Dermatol 136: 619-24, 2000).

Phototherapeutical systems attached to endoscope are used for the photodynamic treatment of tumours (bladder carcinoma or bronchial cancer), however, in these instruments no ultraviolet light is used, and they have special distal ends for tumour treatment (U.S. Patents of Nos. 4,313,431; 4,612,938; 4,676,231; 4,998,930; 5,146,917).

At present, the phototherapeutical systems delivering ultraviolet light consist of a hand piece specifically shaped to a special problem, and they are either unsuitable or little suitable for the treatment of small body cavities such as the nasal cavity with visual control.

We aimed to develop an ultraviolet light emitting phototherapeutical apparatus for the treatment of nasal cavity and paranasal sinuses to construct a special ultraviolet light source.

Only the light of ultraviolet light delivering lasers can be lead with good efficiency into thin optical fibre cables having diameter of a few tenth of millimetre in the most of cases, because of the properties of the laser light, but the laser apparatuses are expensive. It is necessary an ultraviolet light emitting apparatus by this reason, which emits light from a small volume, enabling to lead light energy necessary for the actual phototherapeutic treatment even into an optical fibre cable of small diameter (of a few tenth of millimetre).

It is desirable that this light source should be relatively cheap to manufacture in comparison with the laser ones. One of the potential light sources is a highpressure gas filled lamp with short arch.

Although ultraviolet light has been used for the treatment of hyperproliferative and inflammatory skin diseases since many years, ultraviolet light has not been used for the treatment of common, immunologically mediated disorders of the nasal mucous membrane. Neuman and Finkelstein used narrow-band, low energy, red-light phototherapy for the treatment of the nasal mucous membrane, and they found it effective for perennial allergic rhinitis and for nasal polyposis (Neuman I, Finkelstein Y Narrow-band red light phototherapy in perennial allergic rhinitis and nasal polyposis. Ann Allergy Asthma Immunol 78: 399-406, 1997).

The experimental data underlying the present invention show that single and/or repeated irradiation of the nasal mucous membrane and paranasal sinuses with ultraviolet light of different wavelengths (UVB, UVA, UVB plus UVA, and photochemotherapy with psoralen plus UVA) inhibit the clinical symptoms of rhinitis, sinusitis and rhinosinusitis of different origins, and result the regression of nasal polyps. The constructed apparatus proved to be effective also for the prevention of these diseases, when ultraviolet phototherapy was applied before the appearance of the clinical symptoms.

The subject-matter of this invention is a phototherapeutical apparatus, which is used for the treatment and for the prevention of the diseases, of the nasal cavity and paranasal sinuses.

The apparatus is provided with a source of ultraviolet light, an optical coupling unit focusing the light, a light delivery system and a contact unit of ultraviolet irradiation.

The light source is a quartz bulb filled with a gas or a mixture of gases, e. g. a mixture of xenon, argon and mercury vapour. The gas or mixture of gases is able to emit light also in the ultraviolet spectrum under influence of electric discharge. The bulb is furthermore provided with a concave mirror reflecting a part of ultraviolet light beam and a condensing lens focusing the ultraviolet light. The quartz bulb, the concave mirror, the condensing lens and an outlet opening are mounted into housing, and an optical filter and a light forwarding system are mounted behind the outlet.

The apparatus described in the present invention can be used effectively for the treatment and for the prevention of inflammatory disorders of the nasal cavities, such as in hay fever, vasomotor rhinitis, nonallergic eosinophilic rhinitis, chronic sinusitis and in nasal polyposis.

The subject of the invention is phototherapeutical apparatus for the treatment and for the prevention of diseases of the nasal cavity and paranasal sinuses to deliver an ultraviolet light dose in the range of 20-1000 mJ/cm², the apparatus comprising
A) an ultraviolet light source for issuing an ultraviolet light beam comprising:
   i) a housing containing an electric discharge lamp, a concave mirror, a condensing lens, said housing provided with an output opening for emitting the ultraviolet light beam
   ii) an optical filter for filtering the focused light emitted from the output opening wherein said filter allows to pass UVA and/or UVB light,
      - wherein said electric discharge lamp comprises:

      - a bulb filled with gas emitting light partially in the ultraviolet spectrum;
      - electrodes in said bulb connected to an electric power supply unit;
      - wherein said electric discharge lamp having a discharge volume of some mm³ to some tenth of mm³ and
      - wherein said concave mirror is positioned on one side of the bulb for reflecting a part of the produced ultraviolet light beam;
      - wherein said condensing lens is positioned on the other side of the bulb for focusing the whole ultraviolet light beam;
B) an optical coupling unit for leading the ultraviolet light beam to a light delivery system;
C) a light delivery system consisting of an optical cable having a diameter between 0,001 - 10 mm;
D) an ultraviolet light irradiating contact unit comprising
   - a handgrip and an optical tube mounted thereon wherein the light delivery system connects to the handgrip;
   - a light source built into the handgrip for emitting viewing light;
   - a mirror or a dichroic mirror mounted in one end of said optical tube for projecting the viewing light onto the surface to be illuminated;
   - a magnifying lens mounted on the same end of said optical tube for visual control of the surface to be treated;
   - a tapered head mounted onto the other end of said optical tube, said head has an output window to lead out the ultraviolet light beam and the viewing light together.

The phototherapeutical apparatus according to invention, its structural elements, as well are shown in the Figures 1- 2 to 4-10.

The Figure 1 shows the main elements of the phototherapeutical apparatus and their arrangement.

The Figure 2 shows the ultraviolet light source and the optical coupling unit.

The Figure 4 shows an embodiment of the contact unit for irradiation with ultraviolet light, wherein the light forwarding unit is inserted into the body of the ultraviolet light irradiating contact unit.

The Figure 5 shows an embodiment of the ultraviolet light irradiating contact unit, wherein the body of ultraviolet light irradiating contact unit does not contain any light delivery system. The ultraviolet light is directed by a dichroic mirror, while the scanning light is directed with a concave mirror having a hole in the middle onto the surface to be treated.

The Figure 6 shows an embodiment of the ultraviolet light irradiating contact unit, which ends with a light reflecting surface, and which is applicable for treatment of a flat surface.

The Figure 7 shows an embodiment of the ultraviolet light irradiating contact unit, which ends with a reflecting surface, applicable for cylindrically symmetric treatment.

The Figure 8 shows an embodiment of the ultraviolet light irradiating contact unit, which is applicable for a dot-like treatment.

The Figure 9 shows a phototherapeutical apparatus, which is applicable for a treatment using a flexible endoscop.

In Figure 10 we show that ultraviolet photochemotherapy decreases the clinical scores of the nasal blockage, nasal itch, nose running, sneeze and itching of the palate in patients with allergic rhinitis.

The phototherapeutical apparatus described in the present invention is suited for the treatment and prevention of common inflammatory diseases of the nasal mucous membrane and paranasal sinuses such as allergic rhinitis (hay fever), vasomotor rhinitis, nonallergic eosinophilic rhinitis, chronic sinusitis (inflammation in the paranasal sinuses), or nasal polyps with ultraviolet light.

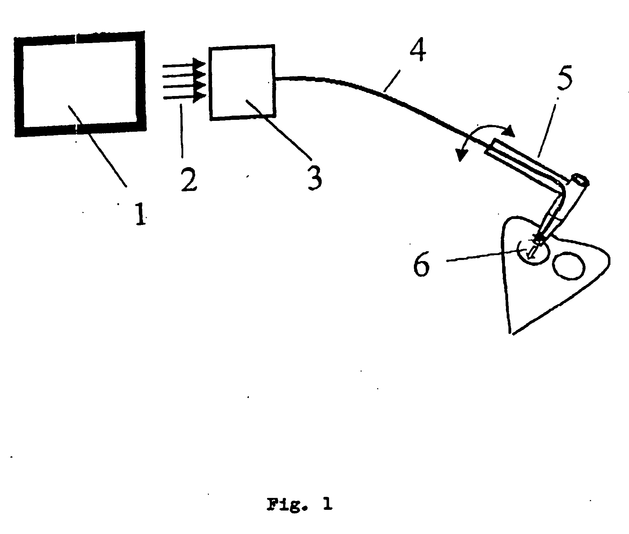

The Figure 1 shows the connection arrangement of the phototherapeutical apparatus applicable to the treatment of nasal mucous membrane.

The Figure 1 shows the gas filled ultraviolet light lamp 1 the outgoing ultraviolet light beam 2, the optical coupling unit 3, wherein the ultraviolet light beam is focused, while it goes through. The light beam enters the light delivery system 4, then goes into the ultraviolet light irradiating contact unit 5. The contact unit is inserted into nostril 6 of the patient. The light is directed from the contact unit onto nasal mucous membrane.

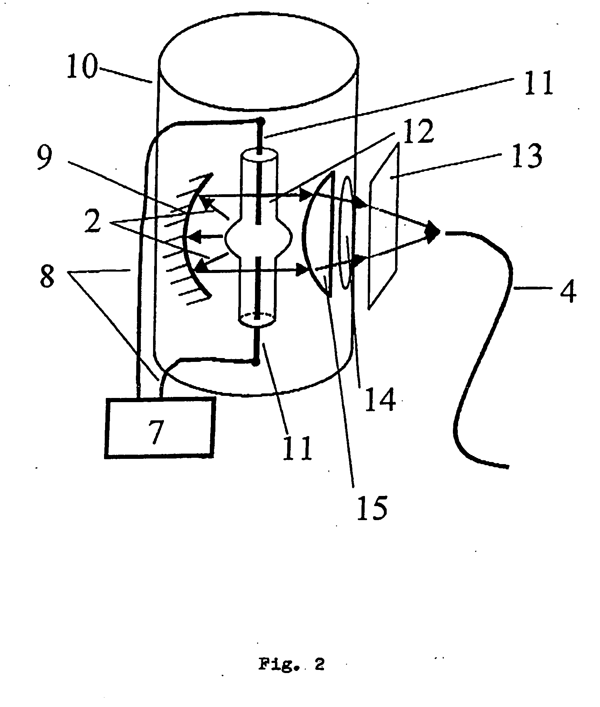

We apply the ultraviolet light source designed according to invention and shown in the Figure 2.

The Figure 2 shows the electric power supply unit 7 and the wires 8 connecting it to the electrodes 11. The electrodes 11 enter the internal space of quartz bulb 12 filled with gas. The ultraviolet light beam 2 generated in the internal space goes partially reflected by the concave mirror 9, partly immediately to the condensing lens 15. The focused ultraviolet light beam 2 goes from the housing 10 through the output opening 14 to the optical filter 13 then to the light delivery system 4.

The Figure 2 shows that electric current flowing from power supply unit 7 through the wires 8 and the electrodes 11 generates the discharge in the quartz bulb 12 filled with gas or gas mixture, consisting of e. g. xenon, argon and mercury vapour, which emits light partially in the ultraviolet spectrum. The volume of the discharge varies in the range from some mm³ to some tenth of mm³. This discharge volume emitting light beam, consisting partially of ultraviolet light is placed in the focus of a concave mirror 9, so a part of the light beam emitted by the discharge goes to the mirror 9, then the reflected parallel beam goes into the condensing lens 15. Another part of the light beam 2 containing ultraviolet light goes immediately to the condensing lens 15. The condensing lens 15 focuses the light rays reaching it. The focused light rays go out of the housing 10 through the output opening 14 onto optical filter 13. The optical filter 13 performs the spectral filtering, of the emitted light. According to choice of the optical filter 13 ultraviolet A and/or ultraviolet B rays go through and enter the light forwarding system.

The ultraviolet light is lead by the optical coupling unit 3 to the light forwarding system 4 connected to the ultraviolet light irradiating contact unit 5 which serves for the treatment.

The light delivery system 4 can be any optical cable or arm suited to lead ultraviolet light. The optical cable can be built up of a multitude of known materials like quartz glass or capillary tubes filled with a liquid conducting ultraviolet light and the internal surface of the tubes are covered with ultraviolet reflecting material. The diameter of cable can vary between 1 micron and 10 mm. The light forwarding system 4 can make also a spectral filtering if necessary.

The treatment of the nasal mucous membrane and paranasal sinuses can be performed using differently shaped ultraviolet light irradiating contact units 5. The unit is placed and positioned in the nostril. The most suited ultraviolet light irradiating contact unit 5 for the treatment can be chosen from the multitude of contact units carried out according to invention.

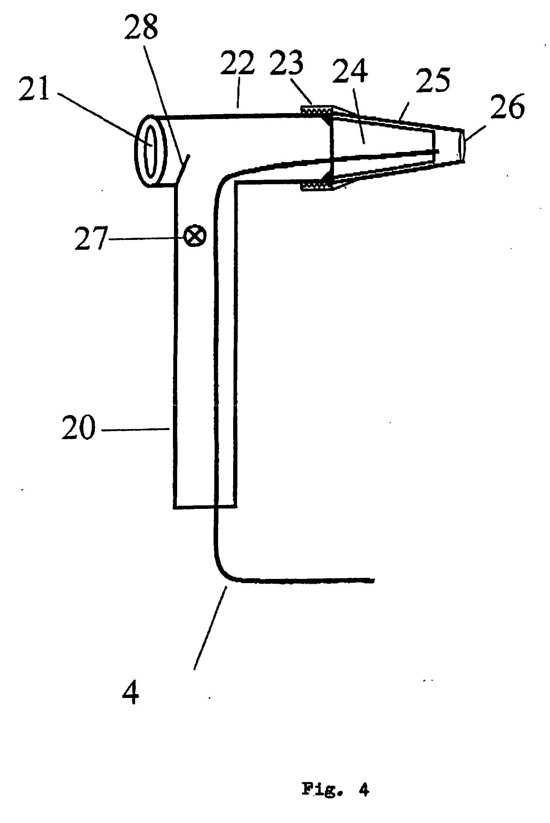

One advantageous embodiment of the ultraviolet light irradiating contact unit 5 is shown in the Figure 4. In this embodiment the light delivery system 4 enters the handgrip 20 goes through the optical tube 22 and ends slightly overhanging in a tapered end piece 24. The head 25 of the UV irradiating contact unit 5 is connected to the optical tube 22 by fastening 23. A light source of scanning light 27 is built into handgrip 20, which can be powered by either an internal or an external power supply unit. Mirror 28 projects the scanning light onto the surface to be illuminated. The ultraviolet light, the scanning light and, if necessary, the targeting light go through the output opening 26 onto surface to be treated. A magnifying glass 21 mounted onto UV irradiating contact unit serves to visual control of the surface being treated with ultraviolet light.

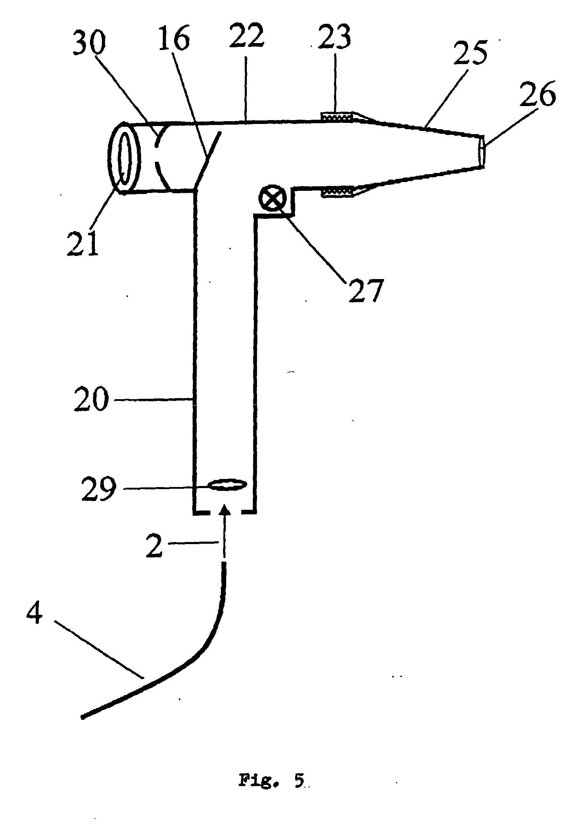

The Figure 5 shows an embodiment of the UVA irradiating contact unit, wherein the ultraviolet light beam 2 goes from the light delivery system 4 to the lens 29 of the handgrip 20, then reflected on the dichroic mirror 16 of optical tube 22 it arrives onto surface to be treated through output opening 26 of the head 25. The scanning light source 27 is mounted into handgrip 20. The scanning light passes the dichroic mirror 16 and reflected by concave mirror with hole 30 illuminates the surface to be treated.

One can apply also UV irradiating contact units 5, wherein the surface treated by ultraviolet light can be controlled using external scanning light.

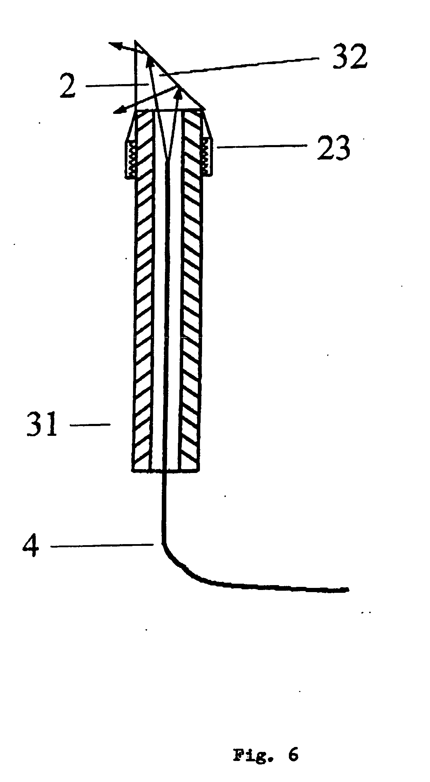

The Figure 6 shows another UV irradiating contact unit wherein the light forwarding system 4 is inserted into a pen-shaped handgrip 31. The ultraviolet light beam 2 is projected by the optical coupling unit 3 into the light delivery system 4, and it arrives into the flat surface treating head 32. The flat surface treating head 32 is coupled to the pen-shaped handle 31 with the fastener 23. The ultraviolet light beam 2 arrives onto surface to be treated after reflection on the surface of a quartz prism or a flat mirror.

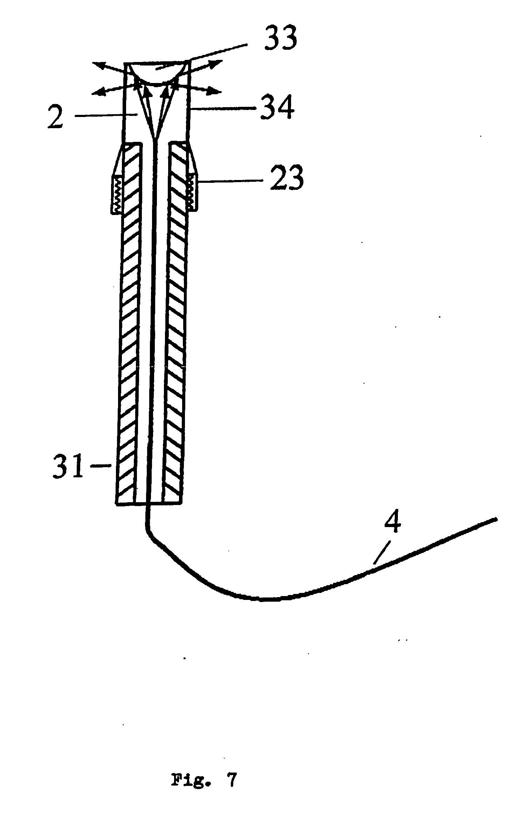

The Figure 7 shows a UV irradiating contact unit 5 suited for the cylindrical symmetric treatment of the nasal cavity. The light delivery system 4 is inserted into the pen-shaped handle 31 in this embodiment. The ultraviolet light beam 2 is lead into the head for cylindrical symmetric treatment 33 of the UV irradiating contact unit 5, and reflected on the conical or spherical reflecting surface it arrives radially onto surface to be treated. The head for cylindrical symmetrical treatment 33 is coupled to the pen-shaped handle 31 with the fastener 23. The nasal cavity can be cylindrically symmetrically treated with this apparatus.

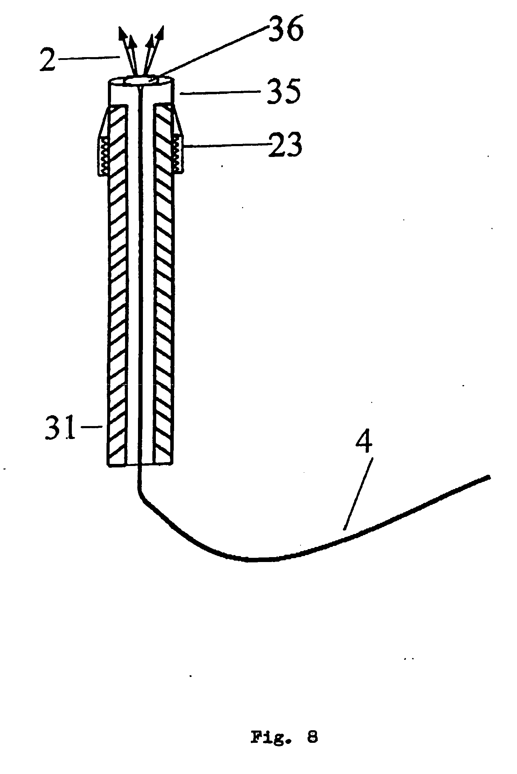

The unit shown in the Figure 8 is suited for dot type treatment of nasal mucous membrane. The light delivery system 4 is inserted into the pen-shaped handle 31. The ultraviolet light beam 2 arrives onto surface to be treated through an end piece 35 for dot type treatment, shaped either as plano-parallel disk or lens 36 made of quartz or plastic transparent to ultraviolet light.

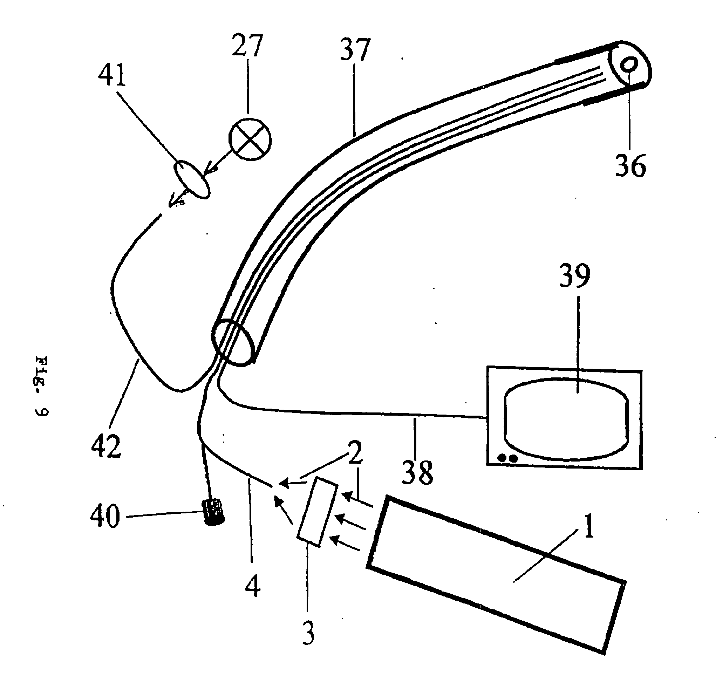

Another unit suited for dot type treatment of the mucous membrane is shown in the Figure 9. The ultraviolet light beam 2 outgoing from the light source 1 is focused in the optical coupling unit 3, forwarded by the light delivery system 4 built into flexible endoscope 37 and arrives to the UV irradiating contact unit 5. The optical cable of the scanner light 42 and the image processing optical cable 38 are mounted into endoscope, as well. A light source 27 of the scanning light supplies the visible light. The lens 41 of scanning light directs the light into the cable of scanning light 42. A plano-parallel disk or a lens 36 covers the end of the endoscope contacting with mucous membrane. The surface treated by ultraviolet light can be visually controlled by the image processing unit 39 of the flexible endoscope 37.

The light forwarding system 4 built into flexible endoscsope 37 can be twisted around its axis by means of the position setting unit 40, so the direction of light beam 2 outgoing from the sloped distal end of light delivery system 4 can be changed. This solution enables also the treatment of other hollow organs, like larynx, digestive canal, urogenital apparatus.

An embodiment of PAL (Panoramic Annular Lens) optical system transparent to the ultraviolet light can be advantageously used instead of the cylindrically symmetric treating head 33 and plano-parallel disk or lens 36 of dot type treatment of the UV irradiating contact unit 5. As the PAL optical apparatus is also suited for optical image processing, it enables the observation of the surface to be treated and the cylindrically symmetric and dot-type treatment at the same time.

The application of the phototherapeutical apparatus described in the present invention is as follows.

The phototherapeutical apparatus described in the present invention is used for the treatment and prevention of diseases of the nasal mucous membrane and paranasal sinuses such as allergic rhinitis (hay fever), vasomotor rhinitis, nonallergic eosinophilic rhinitis, chronic sinusitis (inflammation in the paranasal sinuses), or for the treatment of nasal polyps (a frequent hyperproliferative lesion in chronic inflammatory conditions of the nasal mucous membrane) with ultraviolet light.

These applications are based on our research results, which show that ultraviolet light decreases the number and the activity of inflammatory cells (mast cells, eosinophils, lymphocytes) responsible for the mediator release and synthesis in the mucous membrane, and thereby it reduces the clinical symptoms of allergic and hyperproliferative diseases. This effect of the ultraviolet light is partly due to apoptosis induction.

Before starting the ultraviolet phototherapy of the nose and paranasal sinuses, the light threshold is measured on the skin of the patient previously not exposed to sunlight with the light source applied later for the phototherapy.

The most suited phototherapeutical hand piece for the treatment of mucous membrane of the nose and paranasal sinuses is then chosen, its distal end which contacts to the mucous membrane to be treated is introduced into the nose. The choice of the most suited hand piece depends on the location of the area to be treated, and on the anatomy of the nose and paranasal sinuses of the patient.

The first effective dose for the treatment of nasal mucous membrane with ultraviolet light is between 20 mJ/cm² and 1000 mJ/cm², depending on the applied light source. The treatment with ultraviolet light is repeated once or more times per week using the same dose, or increasing doses, depending on the patient's tolerance and on the improvement of the clinical symptoms.

These treatments based on ultraviolet light inhibit rapidly and significantly the clinical symptoms such as the nasal blockage, nasal itch, nose running, sneeze and itching of the palate in patients with allergic rhinitis.

For the prevention of seasonal allergic rhinitis the ultraviolet phototherapy is started before the appearance of the clinical symptoms. In this case, the treatment is also based on the minimal erythema dose (MED) of the patient, and is given once or several times per week.

To increase the efficacy of the ultraviolet phototherapy, photosensitising substances (for example psoralens) are given before the ultraviolet phototherapy, this is the so-called photochemotherapy. Photosensitising psoralens (5-methoxypsoralen, 8-methoxypsoralen or trimethoxypsoralen) are used in concentrations between 0.0005% and 0.5% in creams or in solutions. When a photosensitising substance (e. g. psoralen) and ultraviolet light is used together for the treatment, the minimal phototoxicitiy dosis (MPD) is measured first on the patient's skin not exposed to sunlight before starting the therapy, this is the smallest ultraviolet dose, which induces erythema on skin after 72 hours. The treatment of the nasal mucous membrane is started depending on the severity of the symptoms of the patient with 0.1 x MPD to 5.0 x MPD, which dose is further increased depending on the patient's tolerance, and repeated once or many times per week.

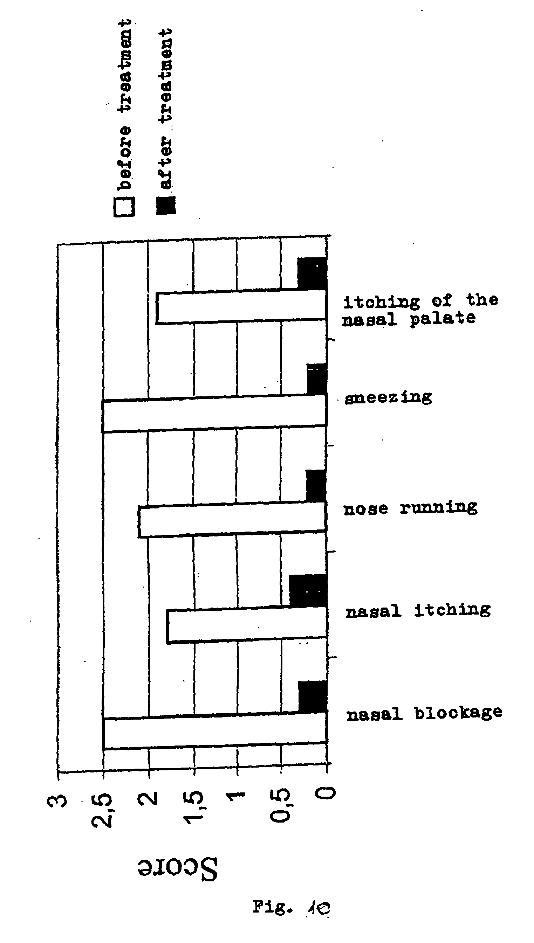

The photochemotherapy inhibits rapidly and effectively the clinical symptoms, such as the nasal blockage, nasal itch, nose running, sneeze and itching of the palate in patients with hay fever. The Figure 10 shows the severity scores of patients with hay fever before photochemotherapy and after a 3-week of ultraviolet photochemotherapy given twice per week with the same doses. All the clinical symptoms and complaints of the patients decreased greatly after the photochemotherapy. Similar improvement is observed after ultraviolet photochemotherapy in the clinical symptoms of vasomotor rhinitis, nonallergic eosinophilic rhinitis, chronic sinusitis, and sizes of nasal polyps considerably decreased.

Similarly to ultraviolet phototherapy, photochemotherapy is also suitable for the prevention of the clinical symptoms of patients with seasonal allergic rhinitis, when the treatment is started before the appearance of the symptoms. For prevention, photochemotherapy is started depending on the severity of the allergy with doses between 0.1 x MPD and 2 x MPD, which are given then once or several times per week depending on the tolerance of the patient.

The phototherapeutical apparatus described in the invention is suitable for the treatment and for the prevention of allergic or autoimmune inflammatory diseases of other organs (gastrointestinal and urogenital tract, conjunctiva).

### Potential side effects

Similarly to that of topical corticosteroides used for the treatment of the disease, ultraviolet light - related to its immunosuppressive effect - might facilitate the appearance of viral and bacterial infections on the treated areas. However, the likelihood for this is lower to that of the presently used local immunity-suppressive preparations, because ultraviolet light has also a direct microbicidal effect (Folwaczny M, Liesenhoff T, Lehn N, Horch HH: Bactericidal action of 308 nm excimer-laser radiation: an in vitro investigation. J Endodontics, 24: 781-785, 1998), and ultraviolet light increases the direct microbicidal activity of epithelial cells (Csató M, Kenderessy SzA, Dobozy A: Enhancement of Candida albicans killing activity of separated human epidermal cells by ultraviolet radiation. Br J Dermatol 116: 469-475, 1987).

It is well known that repeated ultraviolet light irradiation in high doses has carcinogenic potency. The ultraviolet light-induced carcinogen effect is connected with the cumulative dose of the ultraviolet light given in years. As the carcinogenic effect of ultraviolet light depends on its cumulative dose, and the irradiation doses for the phototherapy or photochemotheapy of the nasal mucous membrane are much lower compared to the dosages that increases the risk of cancer; the increased risk for carcinogenesis in the present therapeutical schemes is very low.

The advantages of the phototherapeutical apparatus and of its application are as follows:
For the drug-treatment of common diseases of the nasal mucous membrane and paranasal sinuses such as allergic rhinitis (hay fever), vasomotor rhinitis, nonallergic eosinophilic rhinitis, chronic sinusistis or nasal polyps billions of dollars are expended worldwide. Despite of the available therapeutical opportunities the work-ability of the patients frequently highly decreases. The phototherapeutical apparatus and its application decreases rapidly and effectively the clinical symptoms of the patients, therefore the direct and indirect costs related to the treatment of the diseases decrease in a great extent. Additionally, the ultraviolet light source described in the present invention might be used for the treatment of every known ultraviolet light responsive disease.
The application of the phototherapeutical apparatus according to the invention is shown in the following examples.

### Example 1

The severe symptoms of a patient suffering from raqweed-induced hay fever did not improved satisfactory after using antihistamines and topical corticosteroid nasal drops. At examination the symptoms (nasal blockage, nasal itching, nose running, frequent sneezing and itching of the nasal palate) of the patient were severe, therefore we decided to start photochemotherapy. The minimal phototoxicity dose was measured on the forearm of the patient not exposed to sunlight. The gas filled ultraviolet light lamp 1 shown in the Figure 2 was used for the irradiation in the way that 2 ultraviolet light beams of wavelengths between 310 and 350 nm were lead into light delivery system (optical cable) 4 after usage of optical filters. The MPD was 500 mJ/cm² and the treatment of the nasal mucous membrane was started with a dose of 2.0 x MPD (=1000 mJ/cm²) using the UV irradiating contact unit 5 shown in the Figure 4. Photochemotherapy was performed after using 8-methoxypsoralen nasal spray. The head 25 of the UV irradiating contact unit 5 shown in the Figure 4 was inserted into nasal cavity and the mucous membrane was irradiated with the dose of 1000 mJ/cm². The power density of the ultraviolet light was 43.2 mW/cm² at the output window 26 of the UV irradiating contact unit. The treatment was visually controlled under protection of UV protecting eyeglasses using the visible light beam of the scanning light source 27.

The UV irradiating contact unit 5 was put onto mucous membrane altogether in eight positions in each nostril. The treatment of the nostrils took up approximately 3 minutes and caused no complaints to the patient. This photochemotherapy was repeated once per week. The symptoms of the patient improved considerably already after the second treatment, and after the 3^{rd} treatment the patient was completely free of symptoms. The treatment was stopped after the 4^{th} photochemotherapy, and the symptoms of the patient did not come back.

### Example 2

In a patient with chronic rhinosinusitis with unknown origin the different drugs (antihistamines, corticosteroids, antibiotics) used earlier for the treatment of the symptoms were ineffective, therefore phototherapy was indicated. The ultraviolet light source 1 shown in the Figure 2 was used for the treatment. The minimal erythema dosage (MED) was first determined on the forearm of the patient not exposed to sunlight, then treatment of the nasal mucous membrane was started with a dose of 140 mJ/cm2. The plano-parallel disk 36 of diameter of 4 mm of the UV irradiating contact unit 5 shown in the Figure 4 was brought into contact with the affected mucous membrane and this surface was irradiated for the treatment. The treatment was controlled visually under protection of UV protecting eyeglasses using the visible light of the scanning light source 27. The hand-held apparatus was applied in altogether 8 positions per nostril to irradiate the whole affected mucous membrane. This phototherapy was repeated several times per week. After the 6^{th} treatment the symptoms of the patient decreased considerably, and after the 10^{th} treatment the patient became free of symptoms.

One month after stopping the therapy the patient is still free of symptoms.

### List of references

- 1: Gas filled ultraviolet light lamp
- 2: Ultraviolet light beam
- 3: Optical coupling unit
- 4: Light delivery system
- 5: Ultraviolet light irradiating contact unit
- 6: Nostril
- 7: Electric power supply unit
- 8: Electric wire
- 9: Concave mirror
- 10: Housing
- 11: Electrode
- 12: Quartz bulb
- 13: Optical filter
- 14: Output opening
- 15: Condensing lens
- 20: Handgrip
- 21: Magnifying lens
- 22: Optical tube
- 23: Fastener
- 24: Tapered end piece
- 25: Head
- 26: Output window
- 27: Light source of scanning light
- 28: Mirror
- 29: Lens
- 30: Concave mirror with a hole in the middle
- 31: Pen-shaped handle
- 32: Head for treatment of flat surfaces
- 33: Head for cylindrically symmetric treatment
- 34: Lateral wall transparent for ultraviolet light
- 35: Head for dot-shaped treatment
- 36: Plano-parallel disk or lens
- 37: Flexible endoscope
- 38: Optical cable for image processing
- 39: Image processing unit
- 40: Position setting device
- 41: Lens of scanning light
- 42: Optical cable for scanning light

## Claims

1. A phototherapeutical apparatus for the treatment and for the prevention of diseases of the nasal cavity and paranasal sinuses to deliver an ultraviolet light dose in the range of 20-1000 mJ/cm². the apparatus comprising
A) an ultraviolet light source (1) for issuing an ultraviolet light beam (2) comprising:
i) a housing (10) containing an electric discharge lamp, a concave mirror (9), a condensing lens (15), said housing provided with an output opening (14) for emitting the ultraviolet light beam (2)
ii) an optical filter (13) for filtering the focused light emitted from the output opening (14) wherein said filter allows to pass UVA and/or UVB light,
- wherein said electric discharge lamp comprises:
- a bulb (12) filled with gas emitting light partially in the ultraviolet spectrum;
- electrodes (11) in said bulb connected to an electric power supply unit (7);
- wherein said electric discharge lamp having a discharge volume of some mm³ to some tenth of mm³ and
- wherein said concave mirror (9) is positioned on one side of the bulb (12) for reflecting a part of the produced ultraviolet light beam (2);
- wherein said condensing lens (15) is positioned on the other side of the bulb (12) for focusing the whole ultraviolet light beam (2);
B) an optical coupling unit (3) for leading the ultraviolet light beam (2) to a light delivery system (4);
C) a light delivery system (4) consisting of an optical cable having a diameter between 0,001 - 10 mm;
D) an ultraviolet light irradiating contact unit (5) comprising
- a handgrip (20) and an optical tube (22) mounted thereon wherein the light delivery system (4) connects to the handgrip (20);
- a light source (27) built into the handgrip (20) for emitting viewing light;
- a mirror (28) or a dichroic mirror (16) mounted in one end of said optical tube (22) for projecting the viewing light onto the surface to be illuminated;
- a magnifying lens (21) mounted on the same end of said optical tube (22) for visual control of the surface to be treated;
- a tapered head (24, 25) mounted onto the other end of said optical tube (22), said head (25) has an output window (26) to lead out the ultraviolet light beam (2) and the viewing light together.

## Patentansprüche

1. Ein Apparat der Phototherapie, der für die Pflege und Prävention der Erkrankungen der Nasenhöhle und der Nasennebenhöhlen durch Lieferung einer Dose des ultravioletten Lichtes innerhalb der Grenzen der Leistungsdichte von 20 bis 1000 mJ/cm² bestimmt wird. und er besteht aus
A) einer Quelle des ultravioletten Lichtes (1), die einen ultravioletten Lichtstrahl (2) ausgibt, und sie besteht weiterhin aus:
i) einem Gehäuse (10) das eine Gasentladungsröhre, einen Konkavspiegel (9) und einen Kondensor (15) enthält, und das Gehäuse ist mit einer Ausgangsöffnung (14) zur Emission des ultravioletten Lichtstrahles (2) versehen
ii) einem optischen Filter (13) für Filterung des aus der Ausgangsöffnung (14) emittierten fokussierten Lichtes wobei der Filter das Licht UVA und/oder UVB durchlässt.
- die oben gemeinte Gasentladungslampe besteht aus:
- einer mit einem teilweise in dem ultravioletten Spektrum ausstrahlenden Gas gefüllten Glasbirne (12).
- Elektroden (11), die innerhalb der Glasbirne montiert und mit einer elektrischen Stromquelle (7) verbunden sind:
- die oben gemeinte elektrische Gasentladungslampe hat dabei einen Entladungsraum des Ausmaßes von einigen mm³ bis einigen Zehntels des mm³ und
- der oben gemeinte Konkavspiegel (9) ist dabei auf einer Seite der Glasbirne (12) montiert, um einen Teil des erzeugten ultravioletten Lichtstrahles (2) zu reflektieren.
- der oben gemeinte Kondensor (15) ist dabei auf der entgegengesetzten Seite der Glasbirne (12) montiert, um den ganzen ultravioletten Lichtstrahl (2) zu fokussieren:
B) einer optischen Verbindungseinheit (3), die den ultravioletten Lichtstrahl (2) zum Lichtleitungssystem (4) des ultravioletten Lichtstrahles leitet;
C) einem Lichtleitungssystem (4), das aus einem optischen Kabel besteht, dessen Diameter zwischen 0.001 und 10 mm ist;
D) einer zur Bestrahlung mit ultraviolettem Licht dienenden Kontakteinheit (5), die aus
- einem Handgriff (20) mit einem darauf montierten optischen Rohr (22) dabei das Lichtleitungssystem (4) mit dem Handgriff (20) verbunden ist;
- einer im Handgriff (20) eingebauten den Licht zur Besichtigung emittierenden Lichtquelle (27):
- einem auf einem Ende des gemeinten optischen Rohres (22) montierten Spiegel (28) oder dichroitischen Spiegel (16), der das Licht zur Besichtigung auf die beleuchteten Oberfläche projiziert:
- einer auf dem gleichen Ende des gemeinten optischen Rohres (22) montierten, für die visuelle Besichtigung der behandelten Oberfläche dienenden Vergrößerungslinse (21);
- einem auf dem anderen Ende des gemeinten optischen Rohres (22) montierten konischen Kopf (24. 25), der mit einem Ausgangsfenster (26) versehen ist, um den ultravioletten Lichtstrahl (2) und das den Licht zur Besichtigung zusammen auszuleiten
besteht.

## Revendications

1. Un appareil photothérapeutique destiné à traiter et prévenir les maladies des fosses nasales et des sinuses osseux transmettant une dose des rayons ultraviolets de la densité d'énergie de 20 à 1000 mJ/cm² où cet appareil contient:
A) Une source de la lumière ultraviolet (1) émettante un faisceau des rayons ultraviolets (2) contenant:
i) une boîte (10) contenante une lampe à décharge électrique, un miroir concave (9), un condenseur (15) et cette boîte est munie d'un orifice de sortie (14) pour émettre le faisceau des rayons ultraviolets (2)
ii) un filtre optique (13) pour filtrer la lumière focalisée émise par l'orifice de sortie (14) dans lequel le filtre mentionné laisse passer la lumière UVA et/ou UVB
- la lampe à décharge électique mentionnée contient:
- une ampoule (12) remplie par un gaz émettant la lumière partiellement dans le spectre ultraviolet:
- électrodes (11) dans l'ampoule mentionnée connectées à une source du courant électrique (7):
- la lampe à décharge électrique a un volume de décharge de quelques mm³ à quelques dixièmes de mm³ et
- le miroir concave (9) mentionné est installé à un côté de l'ampoule (12) pour refléter une partie du faisceau des rayons ultraviolets (2) produits:
- le condenseur (15) mentionné est installé au côté opposé de l'ampoule (12) à focaliser entièrement le faisceau des rayons ultraviolets (2):
B) une unité de connexion optique (3) qui dirige le faisceau des rayons ultraviolets (2) à un système de transmission de la lumière (4);
C) un système de transmission de la lumière (4) étant composé d'un câble optique de diamètre de 0.001 à 10 mm;
D) une unité de contact d'irradiation avec la lumière ultraviolet (5) qui contient
- un manche (20) et un tube optique (22) installé sur celui-ci et le système de transmission de la lumière est connecté au manche (20);
- une source de la lumière (27) installée dans le manche (20) qui émet la lumière à regarder:
- un miroir (28) ou un miroir dichroique (16) installé dans un bout du tube optique (22) mentionné qui projette la lumière à regarder sur la surface à éclairer:
- une lentille grossissante (21) installée sur le même bout du tube optique (22) pour le contrôle visuel de la surface à traiter;
- une tête conique (24, 25) installée sur l'autre bout du tube optique mentionné (22), où la tête (25) mentionnée a une fenêtre de sortie (26) pour diriger le faisceau des rayons ultraviolets (2) et la lumière à regarder ensemble.
